(19) Europäisches Patentamt / European Patent Office / Office européen des brevets

(11) **EP 3 445 418 B1**

(12) **EUROPÄISCHE PATENTSCHRIFT**

(45) Veröffentlichungstag und Bekanntmachung des Hinweises auf die Patenterteilung:
**26.01.2022 Patentblatt 2022/04**

(21) Anmeldenummer: **17716550.3**

(22) Anmeldetag: **11.04.2017**

(51) Internationale Patentklassifikation (IPC):
*A61M 1/14* (2006.01)   *A61M 1/16* (2006.01)
*A61M 5/168* (2006.01)

(52) Gemeinsame Patentklassifikation (CPC):
**A61M 1/1613; A61M 1/14;** A61M 1/3639;
A61M 2205/15; A61M 2205/3331; A61M 2205/70

(86) Internationale Anmeldenummer:
**PCT/EP2017/058706**

(87) Internationale Veröffentlichungsnummer:
**WO 2017/182337 (26.10.2017 Gazette 2017/43)**

(54) **MEDIZINISCHE BEHANDLUNGSVORRICHTUNG UND VERFAHREN ZUR ÜBERWACHUNG EINER MEDIZINISCHEN BEHANDLUNGSVORRICHTUNG**

MEDICAL TREATMENT DEVICE AND METHOD FOR MONITORING A MEDICAL TREATMENT DEVICE

DISPOSITIF THÉRAPEUTIQUE MÉDICAL ET PROCÉDÉ POUR LA SURVEILLANCE D'UN DISPOSITIF THÉRAPEUTIQUE MÉDICAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.04.2016 DE 102016004908**

(43) Veröffentlichungstag der Anmeldung:
**27.02.2019 Patentblatt 2019/09**

(73) Patentinhaber: **Fresenius Medical Care Deutschland GmbH**
**61352 Bad Homburg v.d.H (DE)**

(72) Erfinder: **NOACK, Joachim**
**97616 Bad Neustadt (DE)**

(74) Vertreter: **Oppermann, Frank**
**OANDO Oppermann & Oppermann LLP**
**Wilhelminenstrasse 1a**
**65193 Wiesbaden (DE)**

(56) Entgegenhaltungen:
EP-A1- 1 327 457    WO-A2-2008/024908
WO-A2-2012/151077    US-A1- 2013 233 314

**Beschreibung**

Die Erfindung betrifft eine medizinische Behandlungsvorrichtung, die eine Überwachungseinrichtung aufweist, die derart konfiguriert ist, dass die Überwachung auf der Auswertung des Drucks in dem Fluidsystem basiert. Darüber hinaus betrifft die Erfindung ein Verfahren zur Überwachung einer medizinischen Behandlungsvorrichtung, bei dem die Überwachung auf der Auswertung des Drucks in dem Fluidsystem basiert.

Bei einer Dialyse als Beispiel einer extrakorporalen Blutbehandlung durchströmt das zu behandelnde Blut in einem extrakorporalen Blutkreislauf die Blutkammer eines durch eine semipermeable Membran in die Blutkammer und eine Dialysatkammer unterteilten Dialysators, während in einem Dialysierflüssigkeitssystem Dialysat die Dialysatkammer des Dialysators durchströmt. Der extrakorporale Blutkreislauf weist eine Blutzuführleitung auf, die zu der Blutkammer führt, und eine Blutabführleitung auf, die von der Blutkammer abgeht, und das Dialysierflüssigkeitssystem weist eine zu der Dialysatkammer führende Dialysatzuführleitung und eine von der Dialysatkammer abgehende Dialysatabführleitung auf. Im Allgemeinen kann ein Teil des Fluidsystems, das den Dialysator sowie die Blutleitungen umfasst, zur einmaligen Verwendung ausgebildet sein. Die bei einer Blutbehandlung mit Blut befüllten Leitungsabschnitte des Fluidsystems können als Schlauchset und/oder Kassette ausgebildet sein. Das Schlauchset oder die Kassette wird für die Blutbehandlung in die Blutbehandlungsvorrichtung eingelegt bzw. in oder an der Behandlungsvorrichtung befestigt. Das Dialysierflüssigkeitssystem, das die Dialysatleitungen umfasst, kann aber auch als eine austauschbare Einheit (Disposable) ausgeführt sein.

Zum Fördern des Bluts ist eine Blutpumpe im extrakorporalen Blutkreislauf und zum Fördern des Dialysats mindestens eine Dialysatpumpe im Dialysierflüssigkeitssystem vorgesehen. Das frische Dialysat wird in einer Dialysatquelle bereitgestellt und verbrauchtes Dialysat in einen Ablauf abgeführt. Darüber hinaus weisen die Blutbehandlungsvorrichtungen eine Bilanziereinheit zum Bilanzieren von frischem und verbrauchtem Dialysat und eine Ultrafiltrationseinrichtung mit einer Ultrafiltrationspumpe zum Entziehen von Ultrafiltrat aus dem Fluidsystem auf. Es sind auch Dialysevorrichtungen bekannt, bei denen beispielsweise die Bilanziereinrichtung und die Ultrafiltrationseinrichtung zusammengefasst sein können, indem die eingehenden und ausgehenden Flüsse mittels Pumpen gesteuert werden.

Zur Überprüfung der Dichtigkeit eines Fluidsystems, das ein abgeschlossenes Volumen einschließt, sind Druckhaltetests bekannt, bei denen im Fluidsystem ein Über- oder Unterdruck aufgebaut wird. Wenn der Druck in einem vorgegebenen Zeitintervall nicht unter einen bestimmten Grenzwert absinkt oder nicht über einen bestimmten Grenzwert ansteigt, kann darauf geschlossen werden, dass das Fluidsystem dicht ist.

Aus der EP 1 327 457 A1 ist eine Blutbehandlungsvorrichtung bekannt, die über eine Überwachungseinrichtung zur Detektion einer Leckage in dem Fluidsystem der Behandlungsvorrichtung verfügt. Die Überwachungseinrichtung weist einen Drucksensor zum Messen des Drucks im Fluidsystem auf. Während der Blutbehandlung wird der Druck im Fluidsystem fortlaufend gemessen, aus einer möglichen Änderung des Drucks die Leckrate bestimmt, aus der Leckrate das Leckage-Volumen berechnet und das Leckage-Volumen mit einem vorgegebenen Grenzwert verglichen.

Druckänderungen in dem Fluidsystem einer Blutbehandlungsvorrichtung werden auch zur Steuerung der Behandlungsvorrichtung erfasst. Eine sichere Überwachung bzw. Steuerung der Blutbehandlungsvorrichtung setzt daher eine korrekte Auswertung der gemessenen Druckänderungen voraus. In der Praxis hat sich die Auswertung von Druckänderungen zur Überwachung bzw. Steuerung der Behandlungsvorrichtung als nicht unproblematisch erwiesen. Die Festlegung geeigneter Grenzwerte für die Überwachung der Vorrichtung ist in der Praxis schwierig. Denn bei der Festlegung geeigneter Grenzwerte ist zu berücksichtigen, dass die verwendeten Dialysatoren und Schlauchsets oder Kassetten unterschiedliche Eigenschaften haben können, die auf Druckänderungen einen Einfluss ausüben können.

Die WO 2008/024908 A2 beschreibt eine Steuerung für eine medizinische Pumpe zum Fördern von Flüssigkeiten zu einem Patienten. Die Aufgabe der WO 2008/024908 A2 liegt in der genauen Einstellung des mit der Pumpe geförderten Volumens. Zur Steuerung der peristaltischen Pumpe wird die Kraft überwacht, die von den Pumpenelementen auf die Pumpenkammer ausgeübt wird. Folglich befindet sich der Drucksensor nicht in der Pumpenkammer, sondern zwischen Pumpenelement und Pumpenkammer. Ein von der Umgebung abgeschlossenes Volumen, das zumindest Teil eines Flüssigkeitssystems ist, in dem der Druck überwacht wird, ist nicht gegeben.

Die WO 2012/151077 A2 beschreibt eine Infusionsvorrichtung mit einer Pumpe zum Fördern einer Flüssigkeit in einer am Ende offenen Schlauchleitung. Die Infusionsvorrichtung weist einen Drucksensor auf, der den Förderdruck der Pumpe in der Schlauchleitung misst. Wenn der Druck in der Leitung einen vorgegebenen Grenzwert überschreitet, wird ein Alarm gegeben. Die Infusionsvorrichtung erlaubt auch die Bestimmung der Ansprechzeit des Alarmsystems, die dem Benutzer angezeigt wird. Für die Bestimmung der Ansprechzeit wird die Compliance der Schlauchleitung bestimmt.

Die US 2013/0233314 A1 beschreibt ein Beatmungssystem, das eine vom Patienten zu tragende Maske und ein Beatmungsgerät umfasst. Zur Steuerung des Beatmungsgeräts wird in dem System der Druck und die Compliance bestimmt. Die US 2013/0233314 A1 befasst sich mit einem luftgefüllten System, das nicht ein gegenüber der Umgebung abgeschlossenes Volumen bildet.

Der Erfindung liegt die Aufgabe zugrunde, eine medizinische Behandlungsvorrichtung, insbesondere eine extrakorporale Blutbehandlungsvorrichtung, zu schaffen, die eine verbesserte druckbasierte Überwachung ermöglicht. Darüber hinaus ist eine Aufgabe der Erfindung, ein Verfahren anzugeben, das eine verbesserte druckbasierte Überwachung erlaubt.

Die Lösung dieser Aufgaben erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Vorrichtung und das erfindungsgemäße Verfahren erlauben eine verbesserte druckbasierte Überwachung auch dann, wenn unterschiedliche Dialysatoren und/oder Schlauchleitungen zum Einsatz kommen, ohne dass neue Grenzwerte oder Parameter für die Überwachung eingegeben werden müssen.

Die medizinische Behandlungsvorrichtung weist mindestens eine Pumpe zum Fördern von mindestens einer Flüssigkeit in ein und/oder aus einem Fluidsystem auf. Bei der Pumpe kann es sich um eine Blutpumpe zum Fördern von Blut in einem extrakorporalen Blutkreislauf während der Behandlung, eine Dialysatpumpe zum Fördern von Dialysat in einem Dialysierflüssigkeitssystem, eine Pumpe zum Füllen eines Bilanziersystems, eine Ultrafiltrationspumpe oder eine Substitutionspumpe zum Fördern einer Flüssigkeit (Substituat) in das blutseitige Flüssigkeitssystem handeln. Zur Vorbereitung der Blutbehandlung kann die Blutpumpe auch zum Befüllen des Fluidsystems eingesetzt werden. Die Flüssigkeit zum Befüllen des Fluidsystems kann auch in einem Beutel bereitgestellt werden. Bei den Pumpen kann es sich um peristaltische Pumpen handeln, die durch ihre okkludierende Funktion selbst als Absperrorgane dienen können, so dass in dem Fluidsystem ein abgeschlossenes Volumen geschaffen werden kann.

Darüber hinaus verfügt die Behandlungsvorrichtung über eine Überwachungseinrichtung, die derart konfiguriert ist, dass die Überwachung auf der Auswertung des Drucks in dem Fluidsystem, einem Teil des Fluidsystem oder Teilen des Fluidsystems basiert. Die Überwachungseinrichtung kann auch Teil einer Steuereinrichtung zur Steuerung der Behandlungsvorrichtung in Abhängigkeit von dem Druck sein. Zum Messen des Drucks sind ein oder mehrerer Drucksensoren vorgesehen sein. In der Behandlungsvorrichtung können dieser oder diese auch für andere Zwecke vorgesehen sein.

Zur Aufnahme mindestens eines Teils des Fluidsystems kann die medizinische Behandlungsvorrichtung mindestens eine Aufnahme aufweisen, so dass das Fluidsystem oder Teile des Fluidsystems in die Blutbehandlungsvorrichtung eingelegt bzw. an dieser befestigt werden können.

Unter einem Fluidsystem werden sämtliche Bauteile verstanden, die ein Volumen oder mehrere Volumina einschließen, die bestimmungsgemäß beim Betrieb der medizinischen Behandlungsvorrichtung ein oder mehrere Fluide aufnehmen können. Das Volumen oder Teile des Volumens können durch geeignet Organe, beispielsweise Schlauchklemmen, Verschlusskappen etc. gegenüber der Umgebung flüssigkeitsdicht abgeschlossen werden. Das Fluidsystem kann auch mehrerer Teile umfassen, beispielsweise mehrere Schlauchsets oder Kassetten. Wenn das Fluidsystem aus mehreren Teilen besteht, können mehrere Aufnahmen, in die sich die einzelnen Teile einlegen lassen, vorgesehen sein.

Bei einer extrakorporalen Blutbehandlungsvorrichtung kann das Fluidsystem den Dialysator, den extrakorporalen Blutkreislauf und das Dialysierflüssigkeitssystem umfassen. Derartige Fluidsysteme und Aufnahmen gehören zum Stand der Technik. Wenn das Fluidsystem als zur einmaligen Verwendung bestimmtes Schlauchset oder als Kassette konfiguriert sind, kann die Blutbehandlungsvorrichtung mittels Betätigungsorganen auf das Schlauchset oder die Kassette einwirken, beispielsweise Schlauchleitungen an vorgegebenen Stellen des Fluidsystems abklemmen.

Die Behandlungsvorrichtung zeichnet sich durch eine Compliance-Bestimmungseinrichtung zur Bestimmung der Compliance in dem Fluidsystem, einem Teil des Fluidsystem oder Teilen des Fluidsystems aus, wobei die Compliance-Bestimmungseinrichtung mit der Überwachungseinrichtung derart zusammenwirkt, dass die druckbasierte Überwachung in Abhängigkeit von der Compliance des Fluidsystems erfolgt.

In der Praxis hat sich gezeigt, dass die Compliance des Fluidsystems einen nicht zu vernachlässigenden Einfluss auf Druckänderungen ausübt. Daher liegt der Erfindung das Prinzip zugrunde, die Compliance des Fluidsystems, einem Teil des Fluidsystem oder Teilen des Fluidsystems zu bestimmen und die Compliance bei der Festlegung von Grenzwerten bzw. Parametern für die Überwachung der medizinischen Behandlungsvorrichtung zu berücksichtigen. Bei der erfindungsgemäßen Behandlungsvorrichtung kann die Compliance als Auswahlkriterium für die Festlegung von Grenzwerten bzw. Parametern herangezogen werden. Beispielsweise können von der Compliance abhängige Grenzwert- oder Parametersätze für den Betrieb der Behandlungsvorrichtung automatisch ausgewählt werden.

Bei der erfindungsgemäßen Vorrichtung bzw. dem erfindungsgemäßen Verfahren kann eine Änderung der Compliance beispielsweise aufgrund von Luft, die in dem Fluidsystem eingeschlossen sein kann, keinen Einfluss oder einen geringeren Einfluss als ohne Berücksichtigung der Compliance auf die druckbasierte Überwachung ausüben, da die Überwachung in Abhängigkeit von der Compliance erfolgt. Bei einer Blutbehandlungsvorrichtung kann beispielsweise ein nicht entlüfteter Dialysator einen entscheidenden Beitrag zu der Compliance des Systems liefern. In der Praxis kann es sein, dass der Dialysator nicht oder nicht vollständig entlüftet worden ist, wobei sich einzelne Typen von Dialysatoren besser oder schlechter entlüften lassen. Dies ist bei der erfindungsgemäßen Vorrichtung bzw. dem erfindungsgemäßen Verfahren aber nicht von Nachteil, da die Compliance bei der Überwachung Berücksichtigung findet. Die Eigenschaften von unterschiedlichen Schlauchsets oder Kassetten können daher keinen Einfluss auch die Überwachung haben.

Für die Erfindung ist nicht entscheidend, wie die Überwachung der Behandlungsvorrichtung erfolgt. Relevant ist, dass eine druckbasierte Überwachung erfolgt. Insofern kann die Überwachungseinrichtung unterschiedlich konfiguriert sein. Die Compliance-Bestimmungseinrichtung und die Überwachungseinrichtung können als separate Einheiten ausgebildet sein, oder Teil der zentralen Steuer- und Recheneinheit der Behandlungsvorrichtung sein.

Eine Ausführungsform der medizinischen Behandlungsvorrichtung sieht einen Behandlungsmodus und einen der Behandlung vorausgehenden Vorbereitungsmodus zur Vorbereitung der Behandlung vor. Unter einem Behandlungsmodus wird der Betriebszustand der Behandlungsvorrichtung verstanden, in dem die eigentliche Behandlung erfolgt. Bei einer Blutbehandlungsvorrichtung zeichnet sich dieser Betriebszustand dadurch aus, dass der blutseitige Teil des Fluidsystems mit Blut gefüllt ist.

Vor der Inbetriebnahme der Blutbehandlungsvorrichtung ist der blutseitige Teil des Fluidsystems nicht befüllt. Zur Vorbereitung der Blutbehandlung wird der blutseitige Teil des Fluidsystems zunächst mit einer Spülflüssigkeit bzw. einer Vorbereitungsflüssigkeit (häufig als Primingflüssigkeit bezeichnet) befüllt.

Die Überwachungseinrichtung kann derart konfiguriert sein, dass die Bestimmung der Compliance in dem Vorbereitungsmodus und die von der Compliance abhängige, druckbasierte Überwachung in dem Vorbereitungsmodus und/oder dem Behandlungsmodus erfolgt. Dies hat den Vorteil, dass die Bestimmung der Compliance nicht während der Behandlung erfolgt, wenn ein Teil des Fluidsystems mit Blut befüllt ist. Auch braucht die Behandlung nicht unterbrochen zu werden. Wenn der Dialysator einer Blutbehandlungsvorrichtung Teil des Fluidsystems ist, kann die Compliance mit den bekannten Verfahren zudem einfacher bestimmt werden, da ein für die bekannten Verfahren notwendiger Druckausgleich über die Membran des Dialysators im Vorbereitungsmodus, wenn der Dialysator mit Spülflüssigkeit gefüllt ist, schneller erfolgen kann. Die eigentliche druckbasierte Überwachung kann in dem Vorbereitungsmodus vor der und/oder während der Blutbehandlung erfolgen, wozu die Behandlung nicht unterbrochen zu werden braucht. Eine Ausführungsform sieht vor, dass die Überwachungseinrichtung derart konfiguriert ist, dass die von der Compliance abhängige, druckbasierte Überwachung nur in dem Behandlungsmodus erfolgt, und die Compliance in dem Vorbereitungsmodus bestimmt wird.

Die Überwachungseinrichtung kann derart konfiguriert sein, dass das Volumen einer in dem Fluidsystem, einem Teil des Fluidsystems oder Teilen des Fluidsystems eingeschlossenen Flüssigkeit verändert wird, und auf der Grundlage der Druckänderung in dem Fluidsystem, einem Teil des Fluidsystems oder Teilen des Fluidsystems infolge der Änderung des Volumens die Compliance in dem Fluidsystem, einem Teil des Fluidsystems oder Teilen des Fluidsystems bestimmt wird. Dem Fluidsystem, einem Teil des Fluidsystems oder Teilen des Fluidsystems kann hierzu ein vorgegebenes Volumen an Flüssigkeit zugeführt werden oder aus dem Fluidsystem, einem Teil des Fluidsystems oder Teilen des Fluidsystems ein vorgegebenes Volumen an Flüssigkeit abgeführt werden, wobei auf der Grundlage der Druckänderung in dem Fluidsystem, einem Teil des Fluidsystems oder Teilen des Fluidsystems infolge der Flüssigkeitszu- bzw. -abfuhr die Compliance in dem Fluidsystem, einem Teil des Fluidsystems oder Teilen des Fluidsystems bestimmt werden kann. Dabei nehmen die Druckänderungen mit abnehmender Compliance zu bzw. mit zunehmender Compliance ab, d. h. je größer die Nachgiebigkeit bzw. niedriger die Steifigkeit des Systems ist, desto geringer sind die Druckänderungen. Beispielsweise sind die Druckänderungen relativ gering, wenn sich die Wandungen der Schlauchleitungen oder der Kassette bei der Zu- bzw. -abfuhr von Flüssigkeit relativ stark verformen. Wenn aus dem Fluidsystem Flüssigkeit abgeführt wird, braucht der Druck in dem System nicht erhöht zu werden, so dass ein bestimmter Grenzdruck nicht überschritten werden kann. Dabei wird angestrebt, dass die Druckänderung im Bereich des jeweiligen Arbeitspunktes der Behandlungsvorrichtung liegt.

Die Überwachungsvorrichtung kann eine Dichtigkeits-Überwachungseinrichtung sein, die derart konfiguriert ist, dass der Betrag der Änderung des Drucks in einem vorgegebenen Zeitintervall mit einem vorgegebenen Grenzwert verglichen wird, wobei auf eine Undichtigkeit geschlossen wird, wenn der Betrag der Änderung des Drucks größer als der vorgegebene Grenzwert ist. Bei einem Druckabfall um einen vorgegebenen Wert kann beispielsweise in Abhängigkeit von der Compliance auf eine Undichtigkeit geschlossen wird. Die Compliance-Bestimmungseinrichtung wirkt mit der Dichtigkeits-Überwachungseinrichtung derart zusammen, dass der Grenzwert für die Änderung des Drucks in Abhängigkeit von der Compliance festgelegt wird.

Ein Fluidsystem mit einer größeren Compliance beispielsweise erfordert im Allgemeinen die Festlegung eines kleineren Grenzwertes als ein Fluidsystem mit einer kleineren Compliance. Die Festlegung des Grenzwertes kann beispielsweise durch Berechnung des Grenzwertes mit einer die Abhängigkeit des Grenzwertes von der Compliance beschreibenden Gleichung oder die Auswahl eines Grenzwertes aus vorgegebenen Grenzwerten in Abhängigkeit von der Compliance erfolgen. Die Undichtigkeits-Überwachungseinrichtung kann auch derart konfiguriert sein, dass ein Korrekturfaktor für einen bei einem starren Fluidsystem angenommen Grenzwert ermittelt wird, wobei für eine größere Compliance ein kleinerer Korrekturfaktor als für eine kleinere Compliance ermittelt wird.

Bei einer Ausführungsform ist die Compliance-Bestimmungseinrichtung derart konfiguriert, dass das Verhältnis der Compliance in einem ersten Teil des Fluidsystems und der Compliance in einem zweiten Teil des Fluidsystems bestimmt wird, und aus der Compliance in dem ersten Teil des Fluidsystems und dem Verhältnis der Compliance in dem ersten und zweiten Teil des Fluidsystems die Compliance in dem zweiten Teil des Fluidsystems bestimmt wird oder aus der Compliance in dem zweiten Teil des Fluidsystems und dem Verhältnis der Compliance in dem ersten und zweiten Teil des Fluidsystems die Compliance in dem ersten Teil des Fluidsystems bestimmt wird. Dies hat den Vorteil, dass die Compliance nur in einem Teil des Fluidsystems bekannt sein muss. Wenn die Compliance in einem Teil des Fluidsystems bestimmt werden soll, kann die Bestimmung in einem anderen Teil erfolgen, in dem sich die Compliance einfacher und/oder sicherer bestimmen lässt.

Bei dieser Ausführungsform kann die Einrichtung zur Bestimmung der Compliance derart konfiguriert sein, dass das Verhältnis der Compliance in dem ersten und zweiten Teil des Fluidsystems dadurch bestimmt wird, dass bei einem Druckausgleich zwischen dem ersten und zweiten Teil des Fluidsystems das Verhältnis der Druckänderung in dem ersten Teil des Fluidsystems und der Druckänderung in dem zweiten Teil des Fluidsystems bestimmt wird. Ein Vorteil dieser Ausführungsform kann dann zum Tragen kommen, wenn sich die Compliance in dem ersten Teil des Fluidsystems nicht einfach oder sicher bestimmen lässt. Dies kann bei einer extrakorporalen Blutbehandlungsvorrichtung der Fall sein, wenn die Compliance in dem Teil des Fluidsystems bestimmt werden soll, der die Blutkammer des Dialysators sowie die Blutzuführ- und -abführleitung umfasst. Denn die Bestimmung der Compliance sollte nicht in einem mit Blut gefüllten Fluidsystem erfolgen. Die Ausführungsform sieht daher die Bestimmung der Compliance in einem anderen Teil des Fluidsystems der Blutbehandlungsvorrichtung, der ein nicht mit Blut befüllter Teil des Fluidsystems sein kann, insbesondere während der Vorbereitungsphase der Blutbehandlung vor, in der das blutseitige Fluidsystem noch nicht mit Blut befüllt ist. Aus diesem Teil des Fluidsystem, das mit einer Flüssigkeit, beispielsweise Dialysat befüllt sein kann, kann mit der Dialysatpumpe oder der Ultrafiltratpumpe Füssigkeit abgepumpt werden, um eine Druckänderung in dem System hervorzurufen. Das Dialysat kann dann beispielsweise in den Ablauf abgeleitet werden. Die Bestimmung der Compliance zunächst in einem anderen Teil des Fluidsystems kann auch dann von Vorteil sein, wenn sich für die Bestimmung der Compliance nur in dem anderen Teil des Fluidsystems ein bestimmtes Volumen an Flüssigkeit exakt zuführen bzw. abführen lässt.

Im Folgenden wird die Erfindung unter Bezugnahme auf die Figuren im Einzelnen beschrieben.

Es zeigen:

Fig. 1          Komponenten einer Ausführungsform einer erfindungsgemäßen Blutbehandlungsvorrichtung,

Fig. 2          einen schematischen zeitlichen Verlauf des Drucks im Fluidsystem der Blutbehandlungsvorrichtung für den Fall, dass eine Leckage nicht aufgetreten ist, und

Fig. 3          einen schematischen zeitlichen Verlauf des Drucks im Fluidsystem der Blutbehandlungsvorrichtung für den Fall, dass eine geringe Leckage aufgetreten ist,

Fig. 4          ein Ablaufdiagramm mit den einzelnen Phasen der Überwachung für eine Ausführungsform,

Fig. 5          ein weiteres Ausführungsbeispiel einer erfindungsgemäßen Blutbehandlungsvorrichtung und

Fig. 6A und 6B  Schaubilder zur Veranschaulichung der Festlegung von Parametern für die Überwachung der Behandlung.

Die Erfindung wird anhand eines Beispiels einer extrakorporalen Blutbehandlungsvorrichtung beschrieben. Die Erfindung ist aber nicht auf das Ausführungsbeispiel der beschriebenen Blutbehandlungsvorrichtung beschränkt, sondern kann sich auch auf andere Ausführungsformen beziehen, die dem Fachmann bekannt sind. Unter Bezugnahme auf die Figuren sind nur die für die Erfindung relevanten Komponenten der Blutbehandlungsvorrichtung beschrieben. Auch wenn spezielle Ausführungsformen beschrieben sind, so können die Teile auch andere Ausbildungen haben, sofern sie die entsprechende Funktion haben, beispielsweise kann anstelle eines Ventils zum Absperren einer Leitung, eine Klemme oder eine peristaltische Pumpe vorgesehen sein. Ferner müssen nicht alle relevanten Komponenten vorhanden sein.

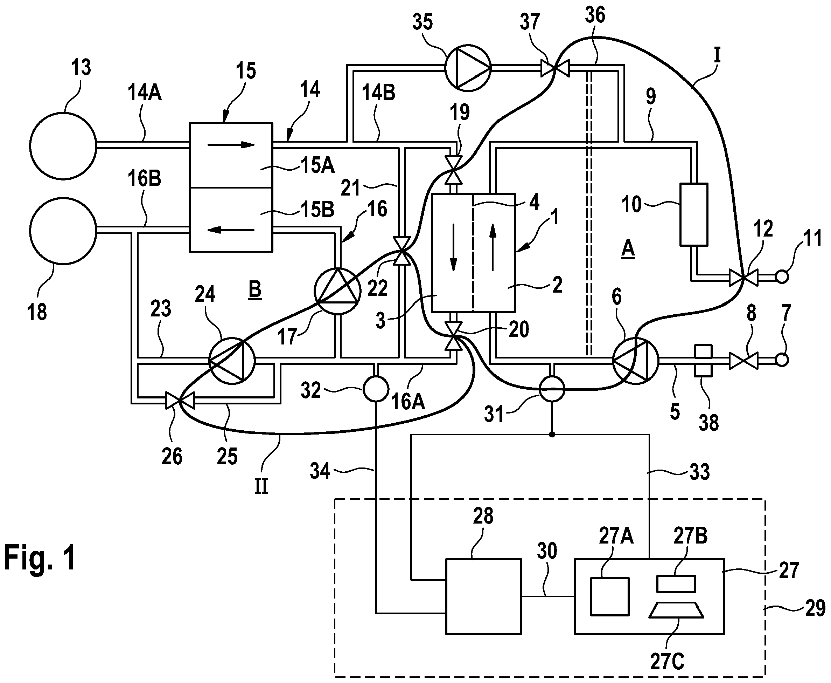

Fig. 1 zeigt die Komponenten einer Blutbehandlungsvorrichtung in stark vereinfachter schematischer Darstellung. Die Blutbehandlungsvorrichtung umfasst einen extrakorporalen Blutkreislauf A und ein Dialysierflüssigkeitssystem B. Der extrakorporale Blutkreislauf A und das Dialysierflüssigkeitssystem B bilden zusammen mit dem Dialysator 1 das Fluidsystem der extrakorporalen Blutbehandlungsvorrichtung. Das Fluidsystem oder Teile des Fluidsystems können als zur einmaligen Verwendung bestimmte Einmalartikel (Disposable) ausgebildet sein. Nachfolgend wird die Blutbehandlungsvorrichtung im aufgerüsteten Zustand beschrieben, in dem das oder die Disposables in die Blutbehandlungsvorrichtung eingelegt bzw. an dieser befestigt sind.

Der Dialysator 1 weist eine Blutkammer 2 und eine Dialysierflüssigkeitskammer 3 auf, die durch eine semipermeable Membran 4 getrennt sind. Eine Blutzuführleitung 5, in der eine Blutpumpe 6 vorgesehen ist, führt von einem arteriellen Patientenanschluss 7 zu dem Einlass der Blutkammer 2. Stromauf der Blutpumpe 6 kann sich in der Blutzuführleitung 5 ein arterielles Absperrorgan 8 befinden. Von dem Auslass der Blutkammer 2 führt eine Blutabführleitung 9, in die eine Flüssigkeitskammer 10 geschaltet sein kann, zu einem venösen Patientenanschluss 11. Stromab der Flüssigkeitskammer 10 befindet sich in der Blutabführleitung 9 ein venöses Absperrorgan 12.

Das Dialysierflüssigkeitssystem B weist eine Dialysatquelle 13 zur Bereitstellung von frischer Dialysierflüssigkeit auf, die über einen ersten Abschnitt 14A einer Dialysatzuführleitung 14 mit dem Einlass einer Kammer 15A einer Bilanziereinheit 15 verbunden ist. Der zweite Abschnitt 14B der Dialysatzuführleitung verbindet den Auslass der einen Kammer 15A der Bilanziereinheit 15 mit dem Einlass der Dialysatkammer 3.

Die Dialysierflüssigkeit kann als Spül- bzw. Vorbereitungsflüssigkeit verwendet werden. Der Auslass der Dialysatkammer 3 ist über einen ersten Abschnitt 16A einer Dialysatabführleitung 16 mit dem Einlass der anderen Kammer 15B der Bilanziereinheit 15 verbunden. In dem ersten Abschnitt 16A der Dialysatabführleitung 16 befindet sich eine Dialysatpumpe 17. Der Auslass der anderen Kammer 15B der Bilanziereinheit 15 ist über den zweiten Abschnitt 16B der Dialysatabführleitung 16 mit einem Abfluss 18 verbunden. Die Bilanziereinheit 15 kann noch weitere Kammern aufweisen.

In der Dialysatzuführleitung 14 befindet sich stromauf der Dialysatkammer 3 ein Absperrorgan 19 und in der Dialysatabführleitung 16 stromab der Dialysatkammer 3 ein Absperrorgan 20. Die Absperrorgane 19, 20 können elektromagnetisch betätigbare Ventile sein. Der zweite Abschnitt 14B der Dialysatzuführleitung 14 kann über eine erste Bypassleitung 21, in der ein erstes Bypassventil 22 vorgesehen ist, mit dem ersten Abschnitt 16A der Dialysatabführleitung 16 verbunden sein. Von dem ersten Abschnitt 16A der Dialysatabführleitung 16 führt eine Ultrafiltratleitung 23, in der eine Ultrafiltratpumpe 24 vorgesehen ist, zu dem zweiten Abschnitt 16B der Dialysatabführleitung 16. Von einem Abschnitt der Ultrafiltratleitung 23 stromauf der Ultrafiltratpumpe 24 kann eine zweite Bypassleitung 25, in der ein zweites Bypass-Ventil 26 vorgesehen ist, zu einem Abschnitt der Ultrafiltratleitung 23 stromab der Ultrafiltratpumpe 24 führen. Die Blutpumpe 6, die Dialysatpumpe 17 und die Ultrafiltratpumpe 24 können okkludierende Pumpen sein. Wenn die Pumpen okkludierende Pumpen sind, können Absperrorgane für die Schaffung eines abgeschlossenen Volumens entfallen.

Die Blutzuführ- und -abführleitung 5, 9 des extrakorporalen Blutkreislaufs A sind bei dem vorliegenden Ausführungsbeispiel Bestandteil eines austauschbaren Blut-Schlauchsets und die Dialysatzuführ- und -abführleitung 14, 16 des Dialysierflüssigkeitssystems B sind fester Bestandteil des Hydraulikteils der Blutbehandlungsvorrichtung. Das BlutSchlauchset sowie der Dialysator 1 sind zur einmaligen Verwendung bestimmt und werden in nicht dargestellte Aufnahmen eingelegt. Anstelle eines Schlauchsets kann auch eine austauschbare Kassette vorgesehen sein, in der Kanäle für die Flüssigkeit, insbesondere die Zufuhr und Abfuhr von Blut, ausgebildet sind. Die Aufnahme für den Dialysator kann eine Halterung sein. In der bzw. an der Blutpumpe 6 können Aufnahmen für die Blutzuführleitung 5 ausgebildet sein.

Die Blutbehandlungsvorrichtung verfügt über eine Überwachungseinrichtung 27 und eine Compliance-Bestimmungseinrichtung 28 zur Bestimmung der Compliance. Die Überwachungseinrichtung 27 und die Compliance-Bestimmungseinrichtung 28 können Bestandteil eine gemeinsame Einrichtung sein, die auch Bestandteil einer zentralen Steuer- und Recheneinrichtung 29 sein können.

Die Überwachungseinrichtung 27 und/oder die Compliance-Bestimmungseinrichtung können beispielsweise einen allgemeinen Prozessor, einen Digitalen Signalprozessor (DSP) zur kontinuierlichen Bearbeitung digitaler Signale, einen Mikroprozessor, eine anwendungsspezifische integrierte Schaltung (ASIC), einen aus Logikelementen bestehenden integrierten Schaltkreis (FPGA) oder andere integrierte Schaltkreise (IC) oder Hardware-Komponenten aufweisen, um die einzelnen Verfahrensschritte auszuführen. Auf den Hardware-Komponenten kann zur Durchführung der Verfahrensschritte ein Datenverarbeitungsprogramm (Software) laufen. Es ist auch eine Mehrzahl oder Kombination der verschiedenen Komponenten möglich.

Die Überwachungseinrichtung 27 und die Compliance-Bestimmungseinrichtung 28 sind über nicht dargestellte Steuer- bzw. Datenleitungen mit den Pumpen 6, 17, 24, den Absperrorganen 8, 12, 19, 20 und den Bypassventilen 22, 26 verbunden, so dass die Flussraten der Pumpen eingestellt und die Absperrorgane und Bypassventile betätigt werden können. Die Absperrorgane und Bypassventile verfügen über nicht dargestellte Betätigungsorgane, die auf das Schlauchset einwirken können.

Die Überwachungseinrichtung 27, die einen Speicher 27A, eine Anzeigeeinrichtung 27B, und eine Alarmeinheit 27C aufweist, ist mit der Compliance-Bestimmungseinrichtung 28 über eine Datenleitung 30 verbunden.

Der Druck im extrakorporalen Blutkreislauf A wird mit einem ersten Drucksensor 31 gemessen, der den Druck in der Blutzuführleitung 5 stromab der Blutpumpe 6 und stromauf der Blutkammer 2 misst. Zusätzlich oder alternativ zu dem Drucksensor 31 an der Blutzuführleitung 5 kann auch ein in Fig. 1 nicht dargestellter Drucksensor an der venösen Blutabführleitung 9, insbesondere stromauf des Absperrorgans 12 vorgesehen sein. Dieser Drucksensor kann zur Bestimmung der Compliance wie der Drucksensor 31 an der Blutzuführleitung 5 herangezogen werden.

Im Dialysierflüssigkeitssystem B wird der Druck mit einem zweiten Drucksensor 32 gemessen, der den Druck in dem ersten Abschnitt 16A der Dialysatabführleitung 16 stromab der Dialysatkammer 3 und stromauf der Dialysatpumpe 17 misst. Es kann auch nur ein Drucksensor vorgesehen sein, der derart angeordnet ist, dass der Druck in dem Bereich bestimmt wird, in dem die Compliance bestimmt wird. Dieser Bereich kann insbesondere den gesamten Dialysator umfassen.

Die Überwachungseinrichtung 27 und die Compliance-Bestimmungseinrichtung 28 empfangen die Signale des ersten Drucksensors 31 über eine Signalleitung 33. Die Compliance-Bestimmungseinrichtung 28 empfängt die Signale des zweiten Drucksensors 32 über eine Signalleitung 34. Die gemessenen Druckwerte werden in der Speichereinheit 27A der Überwachungseinrichtung 27 gespeichert.

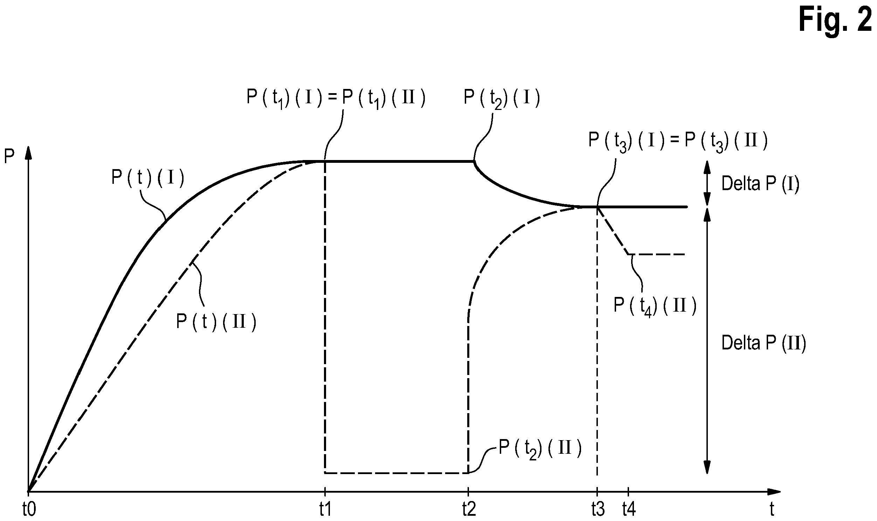

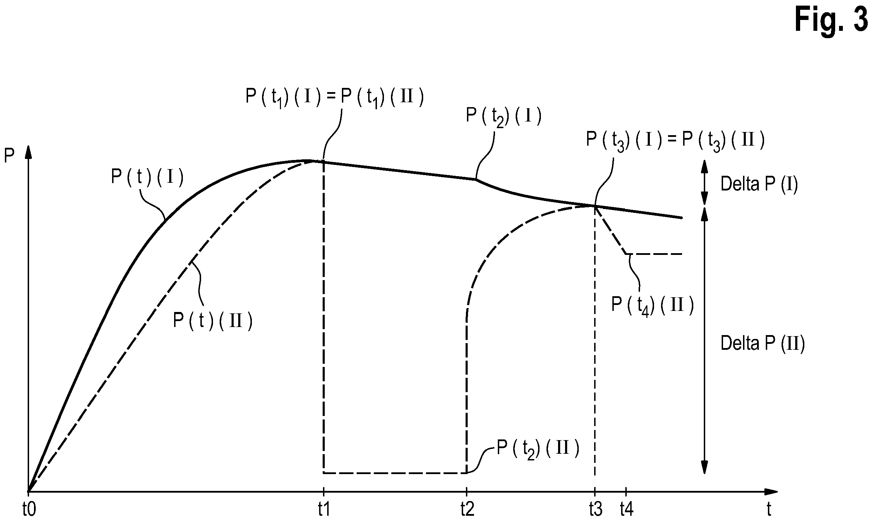

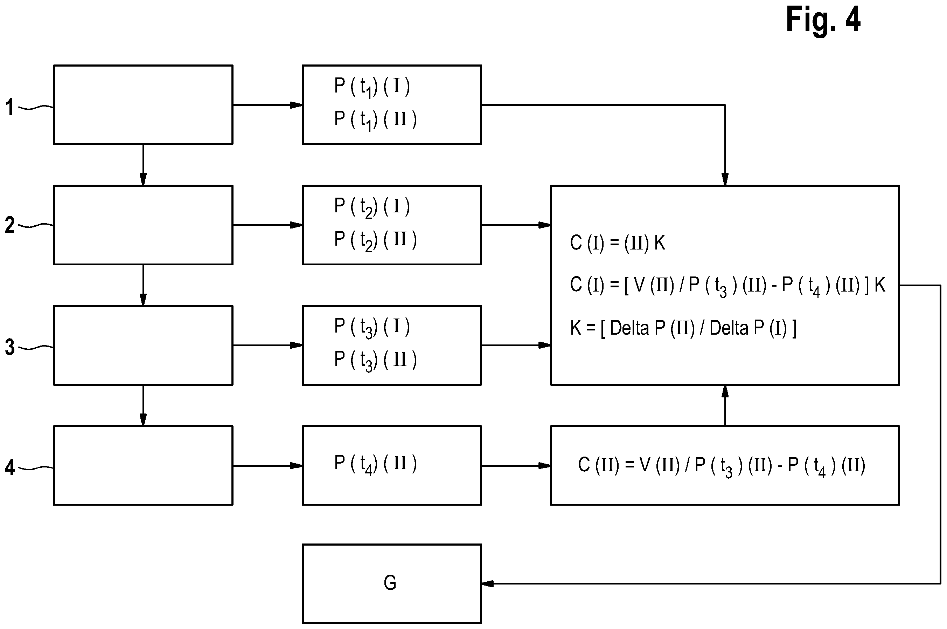

Die Überwachungseinrichtung 27 und die Compliance-Bestimmungseinrichtung 28 steuern die einzelnen Bauteile der Blutbehandlungsvorrichtung wie folgt an, um die nachfolgenden Verfahrensschritte durchzuführen. Die Figuren 2 und 3 zeigen den Druckverlauf. Fig. 4 zeigt ein Ablaufdiagramm mit den einzelnen Phasen.

Bei dem vorliegenden Ausführungsbeispiel ist die Überwachung der Blutbehandlungsvorrichtung die Überprüfung der Dichtigkeit des Fluidsystems. Bei dem vorliegenden Ausführungsbeispiel wird nur ein Teil des Fluidsystems auf Dichtigkeit überprüft. Dieser Teil soll der Teil des Fluidsystems sein, der den Dialysator 1 einschließt. Die Überprüfung der Dichtigkeit des Fluidsystems beruht auf einem Druckhaltetest.

Für die Durchführung des Druckhaltetests wird von dem Fluidsystem der Blutbehandlungsvorrichtung ein mit Flüssigkeit befüllter Teil abgetrennt. Hierzu werden zunächst das arterielle Absperrorgan 12, das Absperrorgan 19 stromauf des Dialysators 1 und das erste und zweite Bypassventil 22, 26 geschlossen, und das zweite Absperrorgan 20 stromab des Dialysators 1 wird geöffnet. Das venöse Absperrorgan 8 braucht nicht geschlossen zu werden, da die Blutpumpe 6 okkludierend ist. Dann wird die Blutpumpe 6 in Betrieb gesetzt, um in dem abgeschlossenen Volumen des Fluidsystems Druck aufzubauen (Phase 1).

Der Überdruck baut sich in dem Abschnitt der Blutzuführleitung 5 zwischen Blutpumpe 6 und Blutkammer 2, in dem Abschnitt der Blutabführleitung 9 zwischen Blutkammer 2 und venösem Absperrorgan 12, in der Blutkammer 2 und der Dialysatkammer 3 und den Leitungsabschnitten zwischen der Dialysatkammer 3 und den Absperrorganen 19, 20 stromauf bzw. stromab der Dialysatkammer 3 auf. Dieser Teil des Fluidsystems wird nachfolgend als erster Teil I des Fluidsystems bezeichnet, in dem der Druckhaltetest durchgeführt werden soll. Da das Absperrorgan 20 stromab der Dialysatkammer 3 geöffnet ist, kann sich ein Überdruck auch in dem Teil des Fluidsystems aufbauen, der die Leitungsabschnitte stromab des Bypassventils 22 und stromauf des Absperrorgans 26 sowie der Dialysatpumpe 17 und der Ultrafiltratpumpe 24 umfasst. Dieser Teil des Fluidsystems wird nachfolgend als zweiter Teil II des Fluidsystems bezeichnet.

Die Blutbehandlungsvorrichtung kann noch über eine Substituatpumpe 35 verfügen, mit der Substituat über eine Substituatleitung 36 dem extrakorporalen Blutkreislauf A stromauf oder stromab der Blutkammer 2 zugeführt werden kann. In der Substituatleitung 36 ist ein weiteres Absperrorgan 37 vorgesehen. Zum Druckaufbau kann anstelle der Blutpumpe 6 auch die Substituatpumpe 35 betrieben werden, die Substituat stromauf oder stromab der Blutkammer der Blutzuführ- bzw. -abführleitung 5, 9 zuführt.

In Fig. 1 sind der erste Teil und zweite Teil des Fluidsystems mit I und II bezeichnet. Der mit dem ersten Drucksensor 31 im ersten Teil I des Fluidsystems zum Zeitpunkt t gemessene Druck wird mit P(t) (I) und der mit dem zweiten Drucksensor 32 im zweiten Teil II des Fluidsystems zum Zeitpunkt t gemessene Druck mit P(t) (II) bezeichnet.

Fig. 2 zeigt den Verlauf des Drucks, der mit dem ersten und zweiten Drucksensor 31, 32 gemessen wird. Dieser Schritt ist in Fig. 4 als Phase 1 bezeichnet. Der mit dem ersten Drucksensor 31 im extrakorporalen Blutkreislauf A gemessene Druck ist mit einer durchgezogenen Linie und der mit dem zweiten Drucksensor 32 im Dialysierflüssigkeitssystem B gemessene Druck mit einer gestrichelten Linie gezeigt. Die Blutpumpe 6 und/oder Substituatpumpe 35 wird zum Druckaufbau so betrieben, dass ein maximal zulässiger Druckgrenzwert und/oder Druckgradient des Dialysators 1 nicht überschritten wird, um eine Schädigung der semipermeablen Membran 4 zu vermeiden. Während des Druckaufbaus ergibt sich eine Druckdifferenz zwischen dem Druck, der mit dem ersten und dem zweiten Drucksensor 31, 32 gemessen wird. Bei sogenannten Highflux-Dialysatoren ist der Flusswiderstand der Membran 4 des Dialysators 1 im Gegensatz zu den sogenannten Lowflux-Dialysatoren zu vernachlässigen. Zum Zeitpunkt t1 ist die Druckdifferenz nahezu Null, da die Blutpumpe 6 relativ langsam läuft. Alternativ oder zusätzlich kann die Vorrichtung konfiguriert sein, eine vorbestimmte Zeit abzuwarten und dann die Druckwerte zu diesem Zeitpunkt zur Bestimmung der Compliance zu verwenden. Alternativ oder zusätzlich kann das System abwarten bis der Gradient der Druckänderung einen vorbestimmten Wert unterschreitet und den Druckwert zu diesem Zeitpunkt zur Bestimmung der Compliance heranziehen. Mit anderen Worten, das System muss nicht zwingend bis zu einem vollständigen Druckausgleich abwarten. Der Druck P(t1) (I) und P(t1) (II) wird gemessen (P(t1) (I) = P(t1) (II)). Es können auch sogenannte Lowflux-Dialysatoren eingesetzt werden, bei denen der Druckausgleich zwischen der Blut- und Dialysierflüssigkeitskammer 2, 3 des Dialysators 1 aber länger dauert, d.h. der Zeitpunkt, zu dem der Druck P(t1) (I) = P(t1) (II) ist später. Die Größe des Druckgradienten hängt auch von der Geschwindigkeit ab, mit der die Flüssigkeit in das bzw. aus dem Fluidsystem gepumpt wird.

Nach erfolgreichem Druckaufbau wird zum Zeitpunkt t1 das Absperrorgan 20 stromab der Dialysatkammer 3 geschlossen, um den ersten Teil I des Fluidsystems von dem zweiten Teil II abzutrennen (Phase 2), so dass der erste und zweite Teil des Fluidsystems jeweils ein abgeschlossenes Volumen bilden. Die Überwachungseinrichtung 27, die bei dem vorliegenden Ausführungsbeispiel eine Dichtigkeits-Überwachungseinrichtung ist, kann nunmehr einen Druckhaltetest in dem ersten Teil I des Fluidsystems durchführen, um diesen Teil des Fluidsystems auf Dichtigkeit zu prüfen, der den Abschnitt der Blutzuführleitung 5 zwischen Blutpumpe 6 und Blutkammer 2, den Abschnitt der Blutabführleitung

7

9 zwischen der Blutkammer 2 und der venösen Schlauchklemme 12, die Blutkammer 2 und die Dialysatkammer 3 des Dialysators 1 sowie die Abschnitte der Dialysatzuführleitung 14 und der Dialysatabführleitung 16 umfasst, an denen sich die nicht dargestellten Kupplungen des Dialysators befinden. Wenn eine Substituatpumpe 35 vorhanden ist, wird auch die Substituatleitung 36 stromab des Absperrorgans 37, beispielsweise des Rückschlagventils, auf Dichtigkeit überprüft.

Wenn der erste Teil I des Fluidsystems dicht ist, bleibt der Druck in diesem Teil des Fluidsystems konstant (P(t1) (I) = P(t2) (I)). Fig. 2 zeigt, den Fall, dass das System dicht ist. Folglich fällt der Druck in dem ersten Teil I des Fluidsystems nicht ab. Fig. 3 zeigt den Fall, dass das System nicht dicht ist. Deutlich ist zu erkennen, dass der Druck in dem ersten Teil I des Fluidsystems abfällt. Eine Undichtigkeit kann dadurch erkannt werden, dass überprüft wird, ob der Druck in einem vorgegeben Zeitintervall unter einen vorgegebenen Grenzwert abfällt oder das Zeitintervall gemessen wird, in dem der Druck um einen vorgegebenen Wert abfällt.

Während des Druckhaltetests in dem ersten Teil I des Fluidsystems wird der zweite Teil II des Fluidsystems belüftet. Hierzu wird das zweite Bypassventil 26 geöffnet, so dass Dialysat in den Ablauf 18 abfließen kann. Der Druck im zweiten Teil II des Fluidsystems fällt somit stark ab. Es stellt sich ein Druck P (II) ein, der größer oder gleich dem Atmosphärendruck ist (Figuren 2 und 3), aber geringer als der Druck im Teil I des Fluidystems ist.

Zu dem Zeitpunkt t2, zu dem die Belüftung des zweiten Teils II des Fluidsystems abgeschlossen ist, wird das zweite Bypassventil 26 wieder geschlossen und das Absperrorgan 20 stromab der Dialysatkammer 3 wieder geöffnet, um zwischen dem ersten und zweiten Teil des Fluidsystems einen Druckausgleich herzustellen (Phase 3). Der Druck im ersten Teil I des Fluidsystems nimmt ab, während der Druck im zweiten Teil II des Fluidsystems zunimmt. Zum Zeitpunkt t3 ist der Druckausgleich abgeschlossen. Zu diesem Zeitpunkt ist der Druck P(t3) (I) gleich dem Druck P(t3) (II).

Die Compliance-Bestimmungseinrichtung 28 wertet den Druckausgleich in dem Zeitraum Δt= t3-t2 wie folgt aus (Fig. 4). Die Compliance-Bestimmungseinrichtung 28 bestimmt den Druckabfall Delta P (I) im ersten Teil I des Fluidsystems aus der Differenz zwischen dem Druck P(t2) (I) zum Zeitpunkt t2 und dem Druck P(t3) (I) zum Zeitpunkt t3. Darüber hinaus bestimmt die Einrichtung 28 die Druckdifferenz Delta P (II) des zweiten Teils II des Fluidsystems aus dem Druck P(t3) (II) zum Zeitpunkt t3 und dem Druck P(t2) (II) zum Zeitpunkt t2. Die Compliance-Bestimmungseinrichtung 28 berechnet nunmehr aus dem Verhältnis der Druckänderungen Delta P das Verhältnis K zwischen der Compliance C (I) in dem ersten Teil I des Fluidsystems und der Compliance C (II) in dem zweiten Teil II des Fluidsystems nach der folgende Gleichung:

$$K = C\ (I)\ /\ C\ (II) =\ Delta\ P\ (II)\ /\ Delta\ P\ (I) \qquad\qquad Gleichung\ (1)$$

Nach erfolgtem Druckausgleich zum Zeitpunkt t3 wird das Absperrorgan 20 stromab der Dialysatkammer 3 wieder geschlossen und die Ultrafiltratpumpe 20 oder die Dialysatpumpe 17 wird kurzzeitig in Betrieb gesetzt, so dass ein bestimmtes Volumen an Dialysat aus dem zweiten Teil II des Fluidsystems abgezogen wird, was einen Druckabfall zur Folge hat. Der Abzug von Dialysat mit der Ultratfiltratpumpe 20 anstelle der Dialysatpumpe 17 hat den Vorteil, dass auch ein definiertes kleines Volumen an Dialysat abgezogen werden kann. Ein bestimmtes Flüssigkeitsvolumen kann auch mit einer bekannten Spritzenpumpe, die sich durch die Förderung kleiner Volumina mit großer Genauigkeit auszeichnet, zugeführt bzw. abgeführt werden. Daher ist auch die Zu- bzw.

Abfuhr von Flüssigkeit mit einer Heparinpumpe möglich, die in Blutbehandlungsvorrichtungen im Allgemeinen bereits vorhanden ist.

Die Verwendung der Ultrafiltratpumpe ist daher geeignet, da diese bestimmungsgemäß während der Behandlung ein sehr definiertes Flüssigkeitsvolumen abpumpen muss und daher bauartveranlasst für das Abpumpen mit wohl definierten Flüssen geeignet ist. Alternativ sind aber Architekturen einsetzbar, die dem Fachmann bekannt sind. So kann auch eine Dialysatpumpe mit einem Flussmesser, beispielsweise mit einem Corioliskraftmesser zum Abpumpen der Flüssigkeit eingesetzt werden. Bei der Ultrafiltratpumpe kann es sich um eine Membranpumpe handeln.

Zum Zeitpunkt t4 stellt sich im zweiten Teil II des Fluidsystems ein Druck P(t4) (II) ein (Phase 4). Die Einrichtung 28 berechnet aus dem bekannten Volumen V (II) des zweiten Teils II des Fluidsystems und der ermittelten Druckdifferenz P(t3) (II) -P(t4) (II) die Compliance C (II) in dem zweiten Teil II des Fluidsystems nach der folgenden Gleichung:

$$C\ (II) = V(II)\ /\ (P(t3)\ (II) - P(t4)\ (II)) \qquad\qquad Gleichung\ (2)$$

Da das Verhältnis K der Compliance zwischen dem ersten und zweiten Teil des Fluidsystems bekannt ist, kann die Einrichtung 28 die Compliance des ersten Teils des Fluidsystems bestimmen. Die Bestimmung der Compliance C erfolgt nach der folgenden Gleichung:

$$C(I) = C(II) K = [V(II) / P(t3)(II) - P(t4)(II)] K \qquad \text{Gleichung (3)}$$

$$K = [Delta\ P\ (II) / Delta\ P\ (I)]$$

Die Überwachungseinrichtung 27 empfängt den Wert der Compliance C (I) über die Datenleitung 30 und führt die Überwachung der Blutbehandlungsvorrichtung in Abhängigkeit von der Compliance durch.

Die Blutbehandlungsvorrichtung kann ferner konfiguriert sein, das oben beschriebene Verfahren auch bei Fluidsystemen mit geringer Leckage, siehe Kurven in Fig. 3, durchzuführen. Bei dieser Ausführungsform kann die Compliance-Bestimmungsvorrichtung konfiguriert sein, aus den zeitlichen Kurvenverläufen durch Extrapolation einen, mehrere oder alle Druckwerte zu den Zeitpunkten t1 bis t4 zu bestimmen. Insbesondere kann dies für den Zeitpunkt t4 erfolgen. Eine solche Extrapolation kann das Ergebnis verbessern, wenn der Druck zum vorbestimmten Zeitpunkt nicht bestimmbar ist. An Beispiel t4 soll dies exemplarisch beschrieben werden: Der Zeitpunkt t4 ist der Zeitpunkt, an dem die Pumpe zum Abziehen des Volumens gestoppt wird. Nun benötigt das System aber eine gewisse Zeit zum Aufnehmen oder Verarbeiten der Druckdaten oder diese werden nur zu bestimmten Zeitscheiben aufgenommen. Erfolgt nun diese Aufnahme nicht exakt zum Zeitpunkt t4, ist dieser Wert nicht bekannt. Fällt zudem wegen einer kleinen Leckage der Druckwert langsam ab, kann der korrekte Druckwert zum Zeitpunkt t4 durch Extrapolation der späteren Druckwerte bestimmt werden, da entweder der Zeitpunkt t4 genau bekannt ist oder der Kreuzungspunkt der abfallenden Kurve zwischen t3 und t4 mit der Kurve nach t4 ermittelt wird.

Für den Druckhaltetest, der von der Überwachungseinrichtung 27 durchgeführt wird, wird ein Grenzwert G festgesetzt. Dieser Grenzwert wird in Abhängigkeit von der Compliance C festgelegt (Fig. 4).

Bei dem vorliegenden Ausführungsbeispiel wird zunächst ein Grenzwert angenommen, der für ein ideales volumenstarres System gilt. Dieser Grenzwert, der in einem Speicher 27A der Steuer- und/oder Überwachungseinrichtung 27 abgespeichert ist, wird mit einem Korrekturfaktor korrigiert, der in Abhängigkeit von der zuvor ermittelten Compliance C (I) bestimmt wird. Beispielsweise können einzelnen Werten der Compliance verschiedene Korrekturfaktoren zugeordnet werden. Diese Korrekturfaktoren werden derart festgelegt, dass mit zunehmender Compliance der angenommene Grenzwert erhöht und mit abnehmender Compliance verringert wird.

Die Überwachungseinrichtung 27 liest den mit dem ersten und zweiten Drucksensor 31, 32 gemessenen Druck P(t1) (I) und P(t2) (I) aus dem Speicher 27A aus, und vergleicht die Druckdifferenz ΔP in dem vorgegebenen Zeitintervall Δt =t2-t1 mit dem in Abhängigkeit von der Compliance C(I) ermittelten Grenzwert G. Wenn der Betrag der Druckdifferenz ΔP kleiner als der Grenzwert ist, wird darauf geschlossen, dass das System dicht ist. Dieser ordnungsgemäße Zustand kann auf der Anzeigeeinrichtung 27B angezeigt werden. Wenn der Betrag der Druckdifferenz ΔP hingegen größer als der Grenzwert ist, d.h. der Druck in dem vorgegebenen Zeitintervall Δt =t2-t1 zu stark abgefallen ist, gibt die Alarmeinheit 27C einen Alarm. In diesem Fall kann die Überwachungseinrichtung 27 auch einen Eingriff in die Maschinensteuerung vornehmen, um die Blutbehandlung zu unterbrechen.

Darüber hinaus berechnet die Überwachungseinrichtung 27 aus dem zuvor gemessenen Druckabfall Δp in dem Zeitintervall Δt =t2-t1 die Leckrate Q nach der folgenden Gleichung:

$$Q(I) = C(I)\ \Delta p/\Delta t \qquad \text{Gleichung (4)}$$

Das Zeitintervall der Δt =t2-t1, in dem die Messwerte aufgenommen werden, kann ein fest vorgegebenes Zeitintervall sein. Es ist aber auch möglich, dass die Änderungsrate dp/dt des Drucks (Steigung der Kurve) erfasst wird, wobei die Messwerterfassung dann erfolgt, wenn das Signal ausreichend stabil ist, d. h. die Druckänderungsrate kleiner als ein Grenzwert ist.

Die Leckrate Q (I) kann auch mit einem vorgegebenen Grenzwert verglichen werden. Wenn die Leckrate größer als der Grenzwert ist, gibt die Alarmeinheit 27C Alarm.

Des Weiteren überprüft die Überwachungseinrichtung 27, ob die Compliance C, in einem Teil I des Fluidsystems, das den Dialysator einschließt, größer als ein vorgegebener Grenzwert ist. Wenn die Compliance C (I) größer als der Grenzwert ist, wird darauf geschlossen, dass der Dialysator 1 nicht ausreichend entlüftet worden ist. Dies kann auf der Anzeigeeinrichtung 27B ebenfalls angezeigt werden.

Bei sämtlichen Ausführungsformen erfolgt die druckbasierte Überwachung in Abhängigkeit von der Compliance C des Fluidsystems, so dass eine sichere Überwachung auch dann möglich ist, wenn sich die Compliance in dem System ändert, beispielsweise infolge von Lufteinschlüssen insbesondere im Dialysator, oder die Compliance des Systems vorab nicht bekannt ist. Die Behandlungsvorrichtung kann derart konfiguriert sein, dass die Compliance im Vorbereitungsmodus und/oder Behandlungsmodus bestimmt wird. Die Anpassung von Grenzwerten oder Parametern kann automatisch erfolgen.

Nachfolgend wird im Einzelnen beschrieben, wann die Bestimmung der Compliance und die Überwachung Behandlungsvorrichtung erfolgen.

Die bekannten Blutbehandlungsvorrichtungen sehen einen Behandlungsmodus und einen der Behandlung vorausgehenden Vorbereitungsmodus zur Vorbereitung der Behandlung vor. Während der Blutbehandlung ist der Teil des Fluidsystems, der die Blutzuführleitung 5, die Blutkammer 2 des Dialysators 1 und die Blutrückführleitung 9 umfasst, mit Blut befüllt, das von der Blutpumpe 6 gefördert wird. In dem der Blutbehandlung vorausgehenden Vorbereitungsmodus ist dieser Teil des Fluidsystems nicht mit Blut befüllt. Die Blutbehandlungsvorrichtung verfügt über eine Einrichtung, die den blutbefüllten Zustand dieses Teils des Fluidsystems erkennen kann. Diese Einrichtung kann ein Blutdetektor 38 sein, der an der Blutzuführleitung 5 vorgesehen ist. Sobald der Blutdetektor 38 Blut erkennt, befindet sich die Blutbehandlungsvorrichtung im Behandlungsmodus. Der Blutdetektor kann ein optischer Detektor sein, der die optische Dichte des Medium im Schlauchsystem bestimmt. Es kann also ausreichen, dass der Blutdetektor Blut von der Vorbereitungsflüssigkeit unterscheiden kann.

Die Überwachungseinrichtung 27 ist derart konfiguriert ist, dass die Bestimmung der Compliance in dem Vorbereitungsmodus und die von der Compliance abhängige, druckbasierte Überwachung in dem Vorbereitungsmodus und/oder dem Behandlungsmodus erfolgt. In dem Vorbereitungsmodus kann das Absperrorgan 12 geschlossen werden und die Blutpumpe 6 braucht nicht betrieben zu werden, so dass der betreffende Teil des Fluidsystems ein abgeschlossenes Volumen bilden kann. In dem Vorbereitungsmodus ist der Dialysator 1 Teil des Fluidsystems, so dass ein den Dialysator einschließender Teil des Fluidsystems überwacht werden kann. Wenn die Compliance im Vorbereitungsmodus bestimmt worden ist, kann die Überwachung im Vorbereitungsmodus oder im Behandlungsmodus erfolgen. Der oben beschriebene initiale Druckhaltetest beispielsweise erfolgt im Vorbereitungsmodus. Weitere von der Compliance abhängige Druckhaltetests können auch im Behandlungsmodus erfolgen. Anstelle eines Druckhaltetests, der nur als ein Beispiel für eine Überwachung beschrieben wird, können in Abhängigkeit von der im Vorbereitungsmodus bestimmten Compliance im Behandlungsmodus beliebige für die Überwachung relevante Parameter der Überwachung festgesetzt oder angepasst werden.

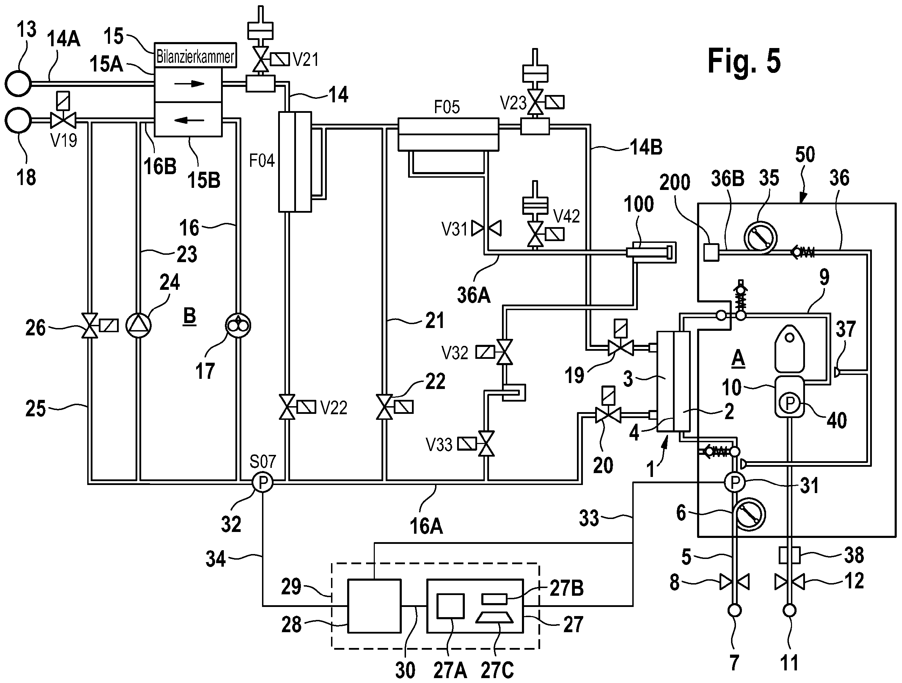

Fig. 5 zeigt ein weiteres Ausführungsbeispiel einer erfindungsgemäßen Blutbehandlungsvorrichtung, bei der der extrakorporale Blutkreislauf A als Einwegkassette 50 ausgebildet ist. In Fig. 5 sind diejenigen Elemente, die denen der Fig. 1 entsprechen, mit denselben Bezugzeichen bezeichnet. Bezüglich dieser Element wird auf eine Wiederholung der Beschreibung verzichtet und auf die Ausführungen zu Fig. 1 verwiesen. Lediglich mögliche Ergänzungen bzw. weitere Ausführungsformen oder Unterschiede zwischen beiden Ausführungsbeispielen werden hier beschrieben.

Die Behandlungsvorrichtung kann wenigstens einen Sterilfilter F04 aufweisen. Fig. 5 zeigt eine Ausführungsform mit zwei Sterilfiltern F04 und F05. Die Sterilfilter dienen dazu, das in der Dialysatquelle 13 bereitgestellte und/oder hergestellte Dialysat weiter zu reinigen. In Fig. 5 ist der maschinenseitige Abschnitt 36A der Substituatleitung 36 noch nicht mit dem kassettenseitigen Leitungsabschnitt 36B verbunden. Für die Fluidverbindung kann in der Behandlungsvorrichtung ein Konnektor 100 vorgesehen sein, der mit einem Konnektor 200 der Einwegkassette zusammenwirken kann.

Darüber hinaus kann die Behandlungsvorrichtung zwischen der Dialysatzuführleitung 14 und der Dialysatabführleitung 16 noch weitere Leitungen aufweisen. Diese in Fig. 5 nicht näher bezeichneten Leitungen können beispielsweise von dem Sterilfilter F04 oder dem Sterilfilter F05 abgehen und zu der Dialysatabführleitung 16 führen.

Für die Bestimmung der Compliance weist die Behandlungsvorrichtung alle notwendigen Verschlusselemente für fluidische Verbindungen auf, um die Volumina, in denen ein Druck aufgebaut wird, abzuschließen. Diese beispielhaft gezeigten Elemente, beispielsweise Ventile, sind in Fig. 5 mit den Bezugzeichen V19, V21, V22, V, 23, V31, V32, V33, V42 versehen.

Im Gegensatz zu separaten Blutschlauchsets verfügt die Einwegkassette 50 für die arterielle Blutzuführleitung 5 und die venöse Blutabführleitung 9 über einen gemeinsamen Gehäusekörper, in dem zumindest Teilstrecken beider Leitungen verlaufen. Ferner kann zumindest auch eine Teilstrecke der Substituatleitung 36 in der Kassette 50 verlaufen. Die Kassette kann auch einen in die venöse Blutleitung 9 mündenden Abzweig der Substituatleitung 36 (Postdilution) und einen in die arterielle Blutleitung 6 mündenden Abzweig der Substituatleitung 36 (Predilution) aufweisen. Ein solches Leitungssystem kann auch bei einem Blutschlauchset verwirklicht sein.

Die Ventile an den Mündungsstellen der Substituatleitung 36 in die venöse und arterielle Blutleitung 5, 9 können in der Kassette ausgebildet sein, beispielsweise das Ventil 37. Als Ventil kann die Kassette eine Folie aufweisen, die von einem in Fig. 5 nicht dargestellten maschinenseitigen Betätigungsorgan gegen einen starren Körper in der Kassette gepresst wird, um einen Fluidkanal zu verschließen. Die Behandlungsvorrichtung kann auch über einen Drucksensor 40 verfügen, der an der venösen Blutleitung 9, insbesondere an der Luftabscheidekammer 10 der venösen Leitung 9 angeordnet ist. Der Druckwert dieses Sensors kann ebenfalls zur Bestimmung der Compliance ausgewertet werden. Zur Messung des Drucks kann die Behandlungsvorrichtung einen Drucksensor aufweisen, der den auf eine Folie der Kassette 50 wirkenden Fluiddruck misst.

Bei einer Kassette, bei der die arterielle und venöse Blutleitung in einem Bauteil zusammengeführt sind, kann es möglich sein, dass sich die für die Entlüftung des Dialysators 1 erforderlichen Maßnahmen nicht durchführen lassen.

Beispielsweise kann es wegen der relativ geringen Länge der Schläuche nicht möglich sein, dass sich der Dialysator zur Entlüftung drehen lässt. Daher kann gerade bei Einwegkassetten die Berücksichtigung der Compliance für die Bestimmung eines oder mehrerer Betriebsparameter der Behandlungsvorrichtung von Bedeutung sein. Damit können beispielsweise die Anzahl von Alarmen und und/oder Fehlermeldungen reduziert werden, womit auch die Nutzungseffizienz, d.h. die Zeit, in der ein Dialysebetrieb stattfinden kann, erhöht werden kann.

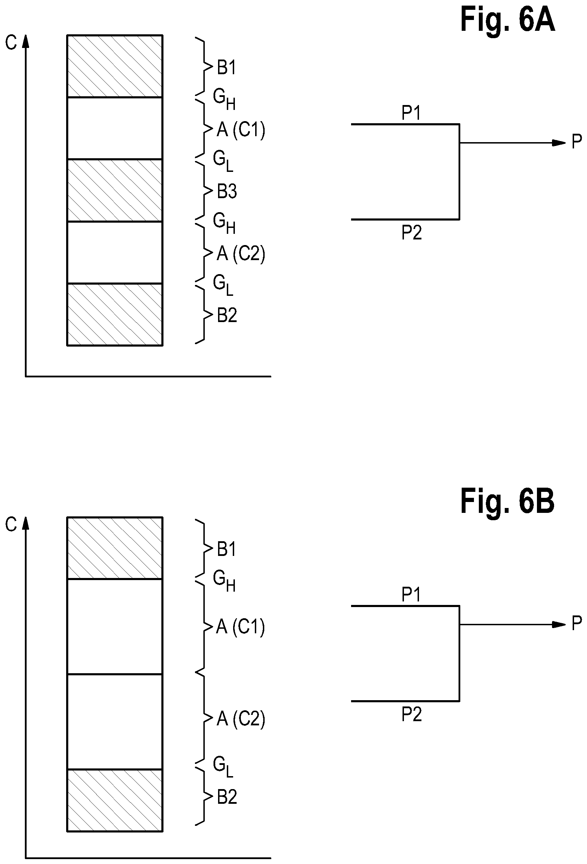

Die Figuren 6A und 6B zeigen Schaubilder zur Veranschaulichung der Festlegung von Parametern für die Überwachung der Behandlung in Abhängigkeit von der Compliance. Bei beiden Ausführungsbeispielen werden in Abhängigkeit von der Compliance jeweils ein oberer und ein unterer Grenzwert $G_H$ und $G_L$ festgelegt. Die Grenzwerte $G_H$ und $G_L$ begrenzen jeweils einen zulässigen Arbeitsbereich A(C1) für eine Compliance C1 bzw. einen zulässigen Arbeitsbereich A(C2) für eine Compliance C2, wobei die Compliance C1 größer als die Compliance C2 ist. Außerhalb der Arbeitsbereiche A(C1) bzw. A(C2) werden obere und untere Alarmbereiche B1, B2, B3 (Fig. 6A) bzw. obere und untere Alarmbereiche B1 und B2 (Fig. 6B) festgelegt.

Fig. 6A veranschaulicht die Auswahl der Parameter für den Fall, dass der obere und untere Arbeitsbereich A(C1) bzw. A(C2) zwei getrennte Bereiche sind, zwischen denen ein Alarmbereich B3 liegt, und Fig. 6B veranschaulicht den Fall, dass der obere und untere Arbeitsbereich A(C1) bzw. A(C2) einen gemeinsamen Arbeitsbereich bilden.

Wenn die Compliance C beispielsweise innerhalb des Alarmbereichs B1 liegt, kann darauf geschlossen werden, dass die Entlüftung des Fluidsystems, insbesondere des Dialysators, nicht ausreichend ist. Liegt die Compliance beispielsweise innerhalb des Alarmbereichs B3, kann darauf geschlossen werden, dass ein nicht bekannter Dialysator verwendet wird. Liegt die Compliance C beispielsweise innerhalb des Alarmbereichs B2, kann darauf geschlossen werden, dass die Bestimmung der Compliance nicht erfolgreich war, da zu erwarten ist, dass die Compliance oberhalb dieses Bereichs liegt. Diese Betriebszustände können auf der Anzeigeeinrichtung 27B angezeigt werden. Innerhalb der Arbeitsbereiche A(C1) bzw. A(C2) kann die Parametrisierung P1 bzw. P2 erfolgen. Beispielsweise kann in Abhängigkeit von der Compliance C ein maximaler Druckabfall $\Delta P1$ bzw. $\Delta P2$ festgelegt werden, der bei einem Druckhaltetest nicht überschritten werden darf, wenn die Dichtigkeit des Fluidsystems angenommen wird. Für den Arbeitsbereich A(C1) wird in Abhängigkeit von der Compliance C für einen größeren Wert der Compliance C1 ein kleinerer maximaler Druckabfall $\Delta P1$ als für einen kleineren Wert der Compliance C2 festgelegt. Alternativ kann auch ein Zeitintervall festgelegt werden, in dem der Druck um einen vorgegebenen Wert abfällt.

**Patentansprüche**

1. Medizinische Behandlungsvorrichtung mit

   mindestens einer Pumpe (6, 17, 24, 35) zum Fördern von mindestens einer Flüssigkeit in ein und/oder aus einem Fluidsystem,
   einer Überwachungseinrichtung (27), die mindestens einen Drucksensor (32, 33) zum Messen des Drucks zumindest in einem Teil des Fluidsystems aufweist, wobei die Überwachungseinrichtung (27) derart konfiguriert ist, dass die Überwachung auf der Auswertung des Drucks in dem Fluidsystem, einem Teil des Fluidsystem oder Teilen des Fluidsystems basiert,
   einer Compliance-Bestimmungseinrichtung (28), die derart konfiguriert ist, das die Compliance in dem Fluidsystem, einem Teil des Fluidsystem oder Teilen des Fluidsystems bestimmt wird, wobei
   die Compliance-Bestimmungseinrichtung (28) mit der Überwachungseinrichtung (27) derart zusammenwirkt, dass die druckbasierte Überwachung in Abhängigkeit von der Compliance des Fluidsystems erfolgt
   **dadurch gekennzeichnet, dass**
   die Überwachungseinrichtung (27) eine Dichtigkeits-Überwachungseinrichtung ist, die derart konfiguriert ist, dass der Druck in dem Fluidsystem, einem Teil des Fluidsystems oder Teilen des Fluidsystems überwacht wird, wobei bei einer Änderung des Drucks in Abhängigkeit von der Compliance auf eine Undichtigkeit des Fluidsystems geschlossen wird.

2. Medizinische Behandlungsvorrichtung nach Anspruch 1, **dadurch gekennzeichnet, dass** die Behandlungsvorrichtung einen Behandlungsmodus und einen der Behandlung vorausgehenden Vorbereitungsmodus zur Vorbereitung der Behandlung vorsieht, wobei die Überwachungseinrichtung (27) derart konfiguriert ist, dass die Bestimmung der Compliance in dem Vorbereitungsmodus und die von der Compliance abhängige, druckbasierte Überwachung in dem Vorbereitungsmodus und/oder dem Behandlungsmodus erfolgt.

3. Medizinische Behandlungsvorrichtung nach Anspruch 2, **dadurch gekennzeichnet, dass** die Überwachungseinrichtung (27) derart konfiguriert ist, dass die von der Compliance abhängige, druckbasierte Überwachung in dem Behandlungsmodus erfolgt.

**4.** Medizinische Behandlungsvorrichtung nach einem der Ansprüche 1 bis 3, **dadurch gekennzeichnet, dass** die Dichtigkeits-Überwachungseinrichtung (27) derart konfiguriert ist, dass der Betrag der Änderung des Drucks in einem vorgegeben Zeitintervall mit einem vorgegebenen Grenzwert verglichen wird, wobei auf eine Undichtigkeit geschlossen wird, wenn der Betrag der Änderung des Drucks größer als der vorgegebene Grenzwert ist, und dass die Compliance-Bestimmungseinrichtung (28) mit der Dichtigkeits-Überwachungseinrichtung (27) derart zusammenwirkt, dass der Grenzwert für die Änderung des Drucks in Abhängigkeit von der Compliance festgelegt wird.

**5.** Medizinische Behandlungsvorrichtung nach einem der Ansprüche 1 bis 4, **dadurch gekennzeichnet**, die Überwachungseinrichtung (27) derart konfiguriert ist, dass das Volumen einer in dem Fluidsystem, einem Teil des Fluidsystems oder Teilen des Fluidsystems eingeschlossenen Flüssigkeit verändert wird, und auf der Grundlage der Druckänderung in dem Fluidsystem, einem Teil des Fluidsystems oder Teilen des Fluidsystems infolge der Änderung des Volumens die Compliance in dem Fluidsystem, einem Teil des Fluidsystems oder Teilen des Fluidsystems bestimmt wird.

**6.** Medizinische Behandlungsvorrichtung nach einem der Ansprüche 1 bis 5, **dadurch gekennzeichnet, dass** die Compliance-Bestimmungseinrichtung (28) derart konfiguriert ist, dass das Verhältnis K der Compliance C (I) in einem ersten Teil (I) des Fluidsystems und der Compliance C (II) in einem zweiten Teil (II) des Fluidsystems bestimmt wird, und aus der Compliance C (I) in dem ersten Teil des Fluidsystems und dem Verhältnis K der Compliance in dem ersten und zweiten Teil des Fluidsystems die Compliance C (II) in dem zweiten Teil (II) des Fluidsystems oder aus der Compliance C (II) in dem zweiten Teil des Fluidsystems und dem Verhältnis K der Compliance in dem ersten und zweiten Teil des Fluidsystems die Compliance C (I) in dem ersten Teil I des Fluidsystems bestimmt wird.

**7.** Medizinische Behandlungsvorrichtung nach Anspruch 6, **dadurch gekennzeichnet, dass** die Einrichtung (28) zur Bestimmung der Compliance derart konfiguriert ist, dass das Verhältnis K der Compliance in dem ersten und zweiten Teil (I, II) des Fluidsystems dadurch bestimmt wird, dass bei einem Druckausgleich zwischen dem ersten und zweiten Teil des Fluidsystems das Verhältnis der Druckänderung in dem ersten Teil des Fluidsystems und der Druckänderung in dem zweiten Teil des Fluidsystems bestimmt wird.

**8.** Medizinische Behandlungsvorrichtung nach einem der Ansprüche 1 bis 7, **dadurch gekennzeichnet, dass** die Behandlungsvorrichtung mindestens eine Aufnahme zur austauschbaren Aufnahme mindestens einen Teils des Fluidsystems aufweist.

**9.** Medizinische Behandlungsvorrichtung nach einem der Ansprüche 1 bis 8, **dadurch gekennzeichnet, dass** die medizinische Behandlungsvorrichtung eine extrakorporale Blutbehandlungsvorrichtung ist, die ein Fluidsystem aufweist, das einen von einer semipermeablen Membran (4) in eine Blutkammer (2) und eine Dialysatkammer (3) unterteilten Dialysator (1), eine zu der Blutkammer (2) führende Blutzuführleitung (5) und eine von der Blutkammer abgehende Blutabführleitung (9), eine zu der Dialysatkammer (3) führende Dialysatzuführleitung (14) und eine von der Dialysatkammer abgehende Dialysatabführleitung (16) aufweist, wobei die Einrichtung (28) zur Bestimmung der Compliance derart konfiguriert ist, dass die Compliance C (I) in einem ersten Teil (I) des Fluidsystems bestimmt wird, der einen Abschnitt der Blutzuführleitung (5) und einen Abschnitt der Blutabführleitung (9) und die Blutkammer (2) und Dialysatkammer (3) des Dialysators (1) umfasst.

**10.** Medizinische Behandlungsvorrichtung nach Anspruch 9, **dadurch gekennzeichnet, dass** die Compliance-Bestimmungseinrichtung (28) derart konfiguriert ist, dass das Verhältnis K der Compliance C (I) in dem ersten Teil (I) des Fluidsystems und der Compliance C (II) in einem zweiten Teil (II) des Fluidsystems, der den ersten Teil des Fluidsystems nicht umfasst, bestimmt wird, und aus der Compliance C (II) in dem zweiten Teil des Fluidsystems und dem Verhältnis K der Compliance in dem ersten und zweiten Teil des Fluidsystems die Compliance C (I) in dem ersten Teil des Fluidsystems bestimmt wird.

**11.** Medizinische Behandlungsvorrichtung nach Anspruch 9 oder 10, **dadurch gekennzeichnet, dass** die medizinische Behandlungsvorrichtung eine Dialysatquelle (13) für frisches Dialysat und einen Ablauf (18) für verbrauchtes Dialysat, eine Blutpumpe (6) zum Fördern von Blut und eine Dialysatpumpe (17) zum Fördern von Dialysat und eine Ultrafiltrationseinrichtung mit einer Ultrafiltrationspumpe (24) zum Entziehen von Ultrafiltrat aufweist, und die Compliance-Bestimmungseinrichtung (28) derart konfiguriert ist, dass aus dem zweiten Teil (II) des Fluidsystems ein vorgegebenes Volumen an Flüssigkeit abgeführt wird, und auf der Grundlage der Druckänderung in dem zweiten Teil des Fluidsystems die Compliance C(II) in dem zweiten Teil (II) des Fluidsystems bestimmt wird, wobei für die Abfuhr von Dialysierflüssigkeit aus dem zweiten Teil des Fluidsystems in den Ablauf (18) die Dialysatpumpe (17) und/oder die Ultrafiltrationspumpe (24) betrieben wird.

**12.** Verfahren zur Überwachung einer medizinischen Behandlungsvorrichtung, die ein Fluidsystem aufweist, das mindestens ein mit einer Flüssigkeit befüllbares Volumens einschließt, bei dem die Überwachung auf der Auswertung des Drucks in dem Fluidsystem, einem Teil des Fluidsystem oder Teilen des Fluidsystems basiert, wobei die Compliance in dem Fluidsystem, einem Teil des Fluidsystems oder Teilen des Fluidsystems bestimmt wird, und dass die druckbasierte Überwachung in Abhängigkeit von der Compliance des Fluidsystems erfolgt, **dadurch gekennzeichnet, dass** der Druck in dem Fluidsystem, einem Teil des Fluidsystems oder Teilen des Fluidsystems überwacht wird, wobei der Betrag der Änderung des Drucks in einem vorgegebenen Zeitintervall mit einem vorgegebenen Grenzwert verglichen wird, wobei auf eine Leckage geschlossen wird, wenn der Betrag der Änderung des Drucks größer als der vorgegebene Grenzwert ist, und dass der Grenzwert für die Änderung des Drucks in Abhängigkeit von der Compliance festgelegt wird.

**13.** Verfahren nach Anspruch 12, **dadurch gekennzeichnet, dass** ein Behandlungsmodus und ein der Behandlung vorausgehender Vorbereitungsmodus zur Vorbereitung der Behandlung vorgesehen ist, wobei die Bestimmung der Compliance in dem Vorbereitungsmodus und die von der Compliance abhängige, druckbasierte Überwachung in dem Vorbereitungsmodus und/oder dem Behandlungsmodus erfolgt.

## Claims

**1.** Medical treatment device comprising

at least one pump (6, 17, 24, 35) for conveying at least one liquid into and/or out of a fluid system, a monitoring apparatus (27) which has at least one pressure sensor (32, 33) for measuring the pressure at least in part of the fluid system, wherein the monitoring apparatus (27) is configured such that monitoring is based on the evaluation of the pressure in the fluid system, part of the fluid system or parts of the fluid system, a compliance-determining apparatus (28) which is configured such that the compliance in the fluid system, part of the fluid system or parts of the fluid system is determined, wherein the compliance-determining apparatus (28) cooperates with the monitoring apparatus (27) in such a manner that the pressure-based monitoring takes place depending on the compliance of the fluid system, **characterised in that** the monitoring apparatus (27) is a tightness-monitoring apparatus which is configured such that the pressure in the fluid system, part of the fluid system or parts of the fluid system is monitored, wherein, where there is a change in the pressure depending on the compliance, it is concluded that the fluid system is not tight.

**2.** Medical treatment device according to claim 1, **characterised in that** the treatment device provides a treatment mode and a preparation mode, preceding the treatment, in preparation for the treatment, wherein the monitoring apparatus (27) is configured such that the determination of the compliance takes place in the preparation mode and the compliance-dependent, pressure-based monitoring takes place in the preparation mode and/or in the treatment mode.

**3.** Medical treatment device according to claim 2, **characterised in that** the monitoring apparatus (27) is configured such that the compliance-dependent, pressure-based monitoring takes place in the treatment mode.

**4.** Medical treatment device according to any one of claims 1 to 3, **characterised in that** the tightness-monitoring apparatus (27) is configured such that the amount of change in the pressure in a specified time interval is compared with a specified limiting value, wherein it is concluded that the system is not tight if the amount of change in the pressure is greater than the specified limiting value, and **in that** the compliance-determining apparatus (28) cooperates with the tightness-monitoring apparatus (27) in such a manner that the limiting value for the change in the pressure is set depending on the compliance.

**5.** Medical treatment device according to any of claims 1 to 4, **characterised in that** the monitoring apparatus (27) is configured such that the volume of a liquid enclosed in the fluid system, part of the fluid system or parts of the fluid system is changed and the compliance in the fluid system, part of the fluid system or parts of the fluid system is determined on the basis of the pressure change in the fluid system, part of the fluid system or parts of the fluid system resulting from the change in volume.

**6.** Medical treatment device according to any of claims 1 to 5, **characterised in that** the compliance-determining apparatus (28) is configured such that the ratio K of the compliance C (I) in a first part (I) of the fluid system and the compliance C (II) in a second part (II) of the fluid system is determined, and the compliance C (II) in the second part (II) of the fluid system is determined from the compliance C (I) in the first part of the fluid system and the ratio K of the compliance in the first and second part of the fluid system, or the compliance C (I) in the first part I of the fluid system is determined from the compliance C (II) in the second part of the fluid system and the ratio K of the compliance in the first and second part of the fluid system.

7. Medical treatment device according to claim 6, **characterised in that** the apparatus (28) for determining the compliance is configured such that the ratio K of the compliance in the first and second part (I, II) of the fluid system is determined by determining the ratio of the pressure change in the first part of the fluid system and the pressure change in the second part of the fluid system in the case of a pressure equalisation between the first and second part of the fluid system.

8. Medical treatment device according to any of claims 1 to 7, **characterised in that** the treatment device has at least one receiver for replaceably receiving at least part of the fluid system.

9. Medical treatment device according to any of claims 1 to 8, **characterised in that** the medical treatment device is an extracorporeal blood-treatment device which has a fluid system comprising a dialyser (1) divided by a semi-permeable membrane (4) into a blood chamber (2) and a dialysate chamber (3), a blood supply line (5) leading to the blood chamber (2) and a blood removal line (9) coming from the blood chamber, a dialysate supply line (14) leading to the dialysate chamber (3) and a dialysate removal line (16) coming from the dialysate chamber, wherein the apparatus (28) for determining the compliance is configured such that the compliance C (I) is determined in a first part (I) of the fluid system that comprises a portion of the blood supply line (5), a portion of the blood removal line (9) and the blood chamber (2) and dialysate chamber (3) of the dialyser (1).

10. Medical treatment device according to claim 9, **characterised in that** the compliance-determining apparatus (28) is configured such that the ratio K of the compliance C (I) in the first part (I) of the fluid system and the compliance C (II) in a second part (II) of the fluid system, which does not include the first part of the fluid system, is determined, and the compliance C (I) in the first part of the fluid system is determined from the compliance C (II) in the second part of the fluid system and the ratio K of the compliance in the first and second part of the fluid system.

11. Medical treatment device according to either claim 9 or claim 10, **characterised in that** the medical treatment device has a dialysate source (13) for fresh dialysate and a drain (18) for used dialysate, a blood pump (6) for conveying blood and a dialysate pump (17) for conveying dialysate, and an ultrafiltration apparatus having an ultrafiltration pump (24) for withdrawing ultrafiltrate, and the compliance-determining apparatus (28) is configured such that a specified volume of liquid is removed from the second part (II) of the fluid system and the compliance C(II) in the second part (II) of the fluid system is determined on the basis of the pressure change in the second part of the system, wherein the dialysate pump (17) and/or the ultrafiltration pump (24) is operated in order to remove dialysis fluid from the second part of the fluid system into the drain (18).

12. Method for monitoring a medical treatment device which has a fluid system which encloses at least one volume which can be filled with a liquid, in which monitoring is based on the evaluation of the pressure in the fluid system, part of the fluid system or parts of the fluid system, wherein the compliance in the fluid system, part of the fluid system or parts of the fluid system is determined, and the pressure-based monitoring takes place depending on the compliance of the fluid system,
**characterised in that**
the pressure in the fluid system, part of the fluid system or parts of the fluid system is monitored, wherein the amount of change in the pressure in a specified time interval is compared with a specified limiting value, wherein it is concluded that there is a leak if the amount of change in the pressure is greater than the specified limiting value, and the limiting value for the change in the pressure is set depending on the compliance.

13. Method according to claim 12, **characterised in that** a treatment mode and a preparation mode, preceding the treatment, in preparation for the treatment are provided, wherein the determination of the compliance takes place in the preparation mode and the compliance-dependent, pressure-based monitoring takes place in the preparation mode and/or the treatment mode.

**Revendications**

1.  Dispositif de traitement médical avec

    au moins une pompe (6, 17, 24, 35) pour le transport d'au moins un liquide dans et/ou depuis un système fluidique, un dispositif de surveillance (27) qui comporte au moins un capteur de pression (32, 33) pour mesurer la pression au moins dans une partie du système fluidique, où le dispositif de surveillance (27) est configuré de telle manière que la surveillance est basée sur l'évaluation de la pression dans le système fluidique, une partie du système fluidique ou des parties du système fluidique,
    un dispositif de détermination de la compliance (28) qui est configuré de telle manière que la compliance dans le système fluidique, une partie du système fluidique ou des parties du système fluidique est déterminée, où le dispositif de détermination de la compliance (28) coopère avec le dispositif de surveillance (27) de telle manière que la surveillance basée sur la pression a lieu en fonction de la compliance du système fluidique **caractérisé en ce que**
    le dispositif de surveillance (27) est un dispositif de surveillance de l'étanchéité qui est configuré de telle manière que la pression dans le système fluidique, une partie du système fluidique ou des parties du système fluidique est surveillée, où lors d'un changement de la pression en fonction de la compliance, on conclut à un défaut d'étanchéité du système fluidique.

2.  Dispositif de traitement médical selon la revendication 1, **caractérisé en ce que** le dispositif de traitement prévoit un mode de traitement et un mode de préparation précédant le traitement pour préparer le traitement, où le dispositif de surveillance (27) est configuré de telle manière que la détermination de la compliance a lieu dans le mode de préparation et la surveillance basée sur la pression dépendante de la compliance a lieu dans le mode de préparation et/ou le mode de traitement.

3.  Dispositif de traitement médical selon la revendication 2, **caractérisé en ce que** le dispositif de surveillance (27) est configuré de telle manière que la surveillance basée sur la pression dépendante de la compliance a lieu dans le mode de traitement.

4.  Dispositif de traitement médical selon l'une des revendications 1 à 3, **caractérisé en ce que** le dispositif de surveillance de l'étanchéité (27) est configuré de telle manière que la valeur du changement de la pression dans un intervalle de temps prédéterminé est comparée à une valeur limite prédéterminée, où on conclut à un défaut d'étanchéité quand la valeur du changement de la pression est supérieure à la valeur limite prédéterminée, et **en ce que** le dispositif de détermination de la compliance (28) coopère avec le dispositif de surveillance de l'étanchéité (27) de telle manière que la valeur limite pour le changement de la pression est établie en fonction de la compliance.

5.  Dispositif de traitement médical selon l'une des revendications 1 à 4, **caractérisé en ce que** le dispositif de surveillance (27) est configuré de telle manière que le volume d'un liquide inclus dans le système fluide, une partie du système fluide ou des parties du système fluide est modifié, et sur la base du changement de pression dans le système fluidique, une partie du système fluidique ou des parties du système fluidique en raison du changement du volume, la compliance dans le système fluidique, une partie du système fluidique ou des parties du fluide système est déterminée.

6.  Dispositif de traitement médical selon l'une des revendications 1 à 5, **caractérisé en ce que** le dispositif de détermination de la compliance (28) est configuré de telle manière que le rapport K de la compliance C (I) dans une première partie (I) du système fluidique et de la compliance C (II) dans une seconde partie (II) du système fluidique est déterminé, et à partir de la compliance C (I) dans la première partie du système fluidique et du rapport K de la compliance dans la première et la seconde partie du système fluidique, la compliance C (II) dans la seconde partie (II) du système fluidique ou à partir de la compliance C (II) dans la seconde partie du système fluidique et du rapport K de la compliance dans la première et la seconde partie du système fluidique, la compliance C (I) dans la première partie I du système fluidique est déterminée.

7.  Dispositif de traitement médical selon la revendication 6, **caractérisé en ce que** le dispositif (28) pour déterminer la compliance est configuré de telle manière que le rapport K de la compliance dans la première et la seconde partie (I, II) du système fluidique est déterminé par le fait que lors d'une égalisation de pression entre la première et la seconde partie du système fluidique, le rapport du changement de pression dans la première partie du système fluidique et du changement de pression dans la seconde partie du système fluidique est déterminé.

**8.** Dispositif de traitement médical selon l'une des revendications 1 à 7, **caractérisé en ce que** le dispositif de traitement comporte au moins un logement pour le logement échangeable d'au moins une partie du système fluidique.

**9.** Dispositif de traitement médical selon l'une des revendications 1 à 8, **caractérisé en ce que** le dispositif de traitement médical est un dispositif de traitement du sang extracorporel qui comporte un système fluidique qui comporte un dialyseur (1) subdivisé par une membrane semi-perméable (4) en une chambre à sang (2) et une chambre à dialysat (3), une conduite d'apport du sang (5) conduisant à la chambre à sang (2) et une conduite d'évacuation du sang (9) partant de la chambre à sang (2), une conduite d'apport du dialysat (14) conduisant à la chambre à dialysat (3) et une conduite d'évacuation du dialysat (16) partant de la chambre à dialysat, où le dispositif (28) pour déterminer la compliance est configuré de telle manière que la compliance C (I) dans une première partie (I) du système fluidique qui comprend une section de la conduite d'apport du sang (5) et une section de la conduite d'évacuation du sang (9) et la chambre à sang (2) et la chambre à dialysat (3) du dialyseur (1) est déterminée.

**10.** Dispositif de traitement médical selon la revendication 9, **caractérisé en ce que** le dispositif de détermination de la compliance (28) est configuré de telle manière que le rapport K de la compliance C (I) dans la première partie (I) du système fluidique et de la compliance C (II) dans une seconde partie (II) du système fluidique, qui ne comprend pas la première partie du système fluidique, est déterminé, et à partir de la compliance C (II) dans la seconde partie du système fluidique et du rapport K de la compliance dans la première et la seconde partie du système fluidique la compliance C (I) dans la première partie du système fluidique est déterminée.

**11.** Dispositif de traitement médical selon la revendication 9 ou 10, **caractérisé en ce que** le dispositif de traitement médical comporte une source de dialysat (13) pour le dialysat frais et un écoulement (18) pour le dialysat usé, une pompe à sang (6) pour le transport du sang et une pompe à dialysat (17) pour le transport du dialysat et un dispositif d'ultrafiltration avec une pompe d'ultrafiltration (24) pour extraire l'ultrafiltrat, et le dispositif de détermination de la compliance (28) est configuré de telle manière qu'un volume prédéterminé de liquide est évacué de la seconde partie (II) du système fluidique, et la compliance C (II) dans la seconde partie (II) du système fluidique est déterminée sur la base du changement de pression dans la seconde partie du système fluidique, où la pompe à dialysat (17) et/ou la pompe d'ultrafiltration (24) est actionnée pour évacuer le liquide de dialyse de la seconde partie du système fluidique dans l'écoulement (18).

**12.** Procédé de surveillance d'un dispositif de traitement médical, qui comporte un système fluidique qui inclut au moins un volume pouvant être rempli avec un liquide, dans lequel la surveillance est basée sur l'évaluation de la pression dans le système fluidique, une partie du système fluidique ou des parties du système fluidique, où la compliance dans le système fluidique, une partie du système fluidique ou des parties du système fluidique est déterminée, et en ce que la surveillance basée sur la pression a lieu en fonction de la compliance du système fluidique, **caractérisé en ce que**
la pression dans le système fluidique, une partie du système fluidique ou des parties du système fluidique est surveillée, où la valeur du changement de la pression dans un intervalle de temps prédéterminé est comparée à une valeur limite prédéterminée, où on conclut à une fuite quand la valeur du changement de la pression est supérieure à la valeur limite prédéterminée, et **en ce que** la valeur limite pour le changement de la pression est déterminée en fonction de la compliance.

**13.** Procédé selon la revendication 12, **caractérisé en ce qu'**il est prévu un mode de traitement et un mode de préparation précédant le traitement pour préparer le traitement, où la détermination de la compliance a lieu dans le mode de préparation et la surveillance basée sur la pression dépendante de la compliance a lieu dans le mode préparation et/ou le mode de traitement.

Fig. 1

Fig. 2

EP 3 445 418 B1

# Fig. 3

Delta P (I)

Delta P (II)

$P(t_3)(I) = P(t_3)(II)$

$P(t_4)(II)$

$P(t_2)(I)$

$P(t_2)(II)$

$P(t_1)(I) = P(t_1)(II)$

$P(t)(I)$

$P(t)(II)$

P

t0   t1   t2   t3   t4   t

# Fig. 4

EP 3 445 418 B1

Fig. 5

# Fig. 6A

C

B1
G_H
A (C1)
G_L
B3
G_H
A (C2)
G_L
B2

P1
P2
P

# Fig. 6B

C

B1
G_H
A (C1)
A (C2)
G_L
B2

P1
P2
P

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1327457 A1
- WO 2008024908 A2
- WO 2012151077 A2
- US 20130233314 A1